# EUROPEAN PATENT APPLICATION

(11) **EP 0 609 978 A1**
(43) Date of publication of application: **10.08.1994**
(21) Application number: 94300099.2
(22) Date of filing: 07.01.1994
(51) Int. Cl.: H01S 3/23, A61B 17/36, A61F 9/00

(54) **Variable repetition rate picosecond laser**

(30) Priority: 04.02.1993 US 13337
(71) Applicant: INTELLIGENT SURGICAL LASERS, INC., San Diego, California 92121-3028 (US)
(72) Inventor: Ujazdowski, Richard C., San Diego, California 92128 (US); Juhasz, Tibor, Irvine, California 92715-4038 (US)
(74) Representative: Coxon, Philip

(57) **Abstract**

A system for varying the pulse repetition rate of a pulsed laser beam includes a computer (10) which is electronically connected to the system's oscillator (12) and regenerative amplifier (14). Specifically, the computer is electronically connected to the oscillator to create a first signal (50) which is indicative of a first pulse repetition rate for the laser beam, as it is initially generated by the oscillator. The computer.then selectively divides the initial first pulse repetition rate by an integer to create a second signal (82) which is indicative of a second pulse repetition rate. The second repetition rate is variable according to the dividing integer and is substantially lower than the first repetition rate. The Pockels cell (62) of the regenerative amplifier is then activated by the computer with the second signal to admit pulses from the oscillator laser beam into the regenerative amplifier at the second pulse repetition rate. Thus, the output laser beam (78) from the regenerative amplifier comprises amplified pulses having a variable second pulse repetition rate. The system may also include an attenuator (84) which is controlled by the computer to establish the energy level of pulses in the output laser beam.

## Description

### FIELD OF THE INVENTION

The present invention pertains generally to systems for varying the physical characteristics of a pulsed laser beam. More specifically, the present invention is useful for electronically varying the repetition rate of pulses in a pulsed laser beam. The present invention is particularly, but not exclusively, useful for electronically controlling and varying the pulse repetition rate of a pulsed laser beam during ophthalmic surgical operations.

### BACKGROUND OF THE INVENTION

It is well known that many systems have been designed and manufactured which are capable of generating either a continuous laser beam or a pulsed laser beam. It has been determined, however, that a pulsed laser beam is preferable for many applications. This is particularly so for applications involving ophthalmic laser surgery. For example, U.S. Patent NO. 4,764,930 which issued to Bille et al. for an invention entitled "Multiwavelength laser source", and which is also assigned to the assignee of the present invention, discloses a source for a pulsed laser beam in which the duration of each pulse is in the picosecond range. Further, examples of ophthalmic surgical applications which effectively use a pulsed laser beam are disclosed in U.S. Patent No. 4,907,586 which issued to Bille et al. for an invention entitled "Method for Reshaping the Eye", and which is also assigned to the assignee of the present invention.

The primary benefit derived from the use of pulsed laser beams in ophthalmic surgery is that, while each pulse may have an otherwise inappropriately high energy level, the pulse can be of very short duration. Consequently, the total energy delivered to the tissue will be very significantly less than what would be delivered by a continuous laser beam having the same energy level. Furthermore, and perhaps more importantly, a pulsed laser beam has quiet intervals between the pulses wherein the tissue can recover from unwanted transient effects of the impact of a laser pulse. Specifically, it is known that when a laser pulse impacts on tissue, in addition to photoablation of the target tissue, the surrounding tissue is subjected to both thermal and mechanical side effects. If left unchecked, these side effects can be very adverse. In fact, they may be so much so that the desired results are unattainable. In summary, pulsed laser beams are preferable for many ophthalmic laser surgery applications, but the energy in the pulses and the repetition rate of the pulses should be controlled.

As is well known in the pertinent art, pulsed laser beams are typically generated by a system having an oscillator and a regenerative amplifier. Specifically, a mode locked oscillator is used to initially generate a beam of pulses which have extremely low energy levels and which have an extremely high pulse repetition rate. For example, oscillator's known in the art will normally generate laser beams which have power levels of around twenty milliwatts (20 mW) and pulse repetition rates on the order of one hundred sixty million Hertz (160 MHz). In order to make such a pulsed beam useable for ophthalmic laser surgery, the energy level needs to be raised. And, very definitely, the pulse repetition rate needs to be lowered. As is well known, the energy level in each pulse of a pulsed beam can be raised by using a regenerative amplifier. Furthermore, as has been recognized by the present invention, the pulse repetition rate of a pulsed beam can be electronically varied by properly controlling the rate at which pulses from the oscillator are admitted to the regenerative amplifier.

A regenerative amplifier includes both a Pockels cell and a pumped laser medium which are mounted in the cavity of the regenerative amplifier. Specifically, the pockels cell is used to admit or introduce laser pulses into the cavity of the regenerative amplifier and the pumped laser medium is used to amplify laser pulses while they are in the cavity. As a practical matter, only one pulse can be amplified at a time. Further, the amplification of the laser pulse depends entirely on the level to which the laser medium has been pumped before the laser pulse is admitted into the cavity by the pockels cell.

Unfortunately, it takes time for the laser medium in a regenerative amplifier to be pumped to its maximum level. Thus, if laser pulses are admitted into the cavity of the regenerative amplifier at a high repetition rate, the laser medium will not have had sufficient time to regenerate itself to its highest possible energy level. The result is that the output pulses from the regenerative amplifier, though having a high repetition rate, will have limited energy levels. The present invention recognizes that a pulse repetition rate below approximately four hundred Hertz (400 Hz) gives the laser medium in the regenerative amplifier sufficient time to regenerate. to its highest energy level.

It may happen, however, that even though it is desirable to have a low pulse repetition rate (e.g. below 400 Hz), the energy level in each pulse may be too high. Accordingly the present invention has recognized that the energy level in individual pulses may need to be controllably attenuated. This is particularly so at the lower pulse repetition rates below 400 Hz.

In light of the above it is an object of the present invention to provide a computer-controlled variable repetition rate picosecond laser system which allows for increasing the energy level in a laser pulse by variably lowering the pulse repetition rate. Another object of the present invention is to provide a computer-controlled variable repetition rate picosecond laser system which can effectively limit the energy level in individual pulses of a pulsed laser beam having a selected pulse repetition rate. Still another object of the present invention is to provide a computer-controlled variable repetition rate picosecond laser system which is controllable by the operator to adjust the pulse repetition rate and the energy level in individual pulses of the output laser beam to accommodate the particular requirements of an ophthalmic laser surgery procedure. Yet another object of the present invention is to provide a computer-controlled variable repetition rate picosecond laser system which is adaptable for use with existing laser systems, is easy to use, and comparatively cost effective.

### SUMMARY OF THE INVENTION

A system for varying the pulse repetition rate of a pulsed laser beam includes a computer which is electronically connected to the oscillator and to the regenerative amplifier of a laser beam generating apparatus. Specifically, the computer uses the pulse repetition rate established by the oscillator as a base signal for selectively creating another signal which activates the operation of the regenerative amplifier to vary the pulse repetition rate of the resultant output laser beam.

For the system of the present invention, the computer receives a first signal from the oscillator which is indicative of the pulse repetition rate of the laser beam that is generated by the oscillator. This first signal is then divided by electronic means in the computer to create a second signal which will establish a second pulse repetition rate. More specifically, the second pulse repetition rate (second signal) is established by electronically dividing the first pulse repetition rate (first signal) with an integer. For purposes of the present invention, first pulse repetition rate will be on the order of one hundred sixty megahertz (160 MHz) and the integer will be equal to or greater than four hundred.

The computer is electronically connected to the pockels cell of the system's regenerative amplifier in order to activate the pockels cell with the second signal. Consequently, the pockels cell admits pulses from the oscillator generated laser beam into the regenerative amplifier at the variably selected second pulse repetition rate. Each pulse admitted into the regenerative amplifier is then subsequently dumped from the regenerative amplifier at the second pulse repetition rate, after being amplified in the regenerative amplifier. Thus, the output laser beam is generated.

According to the present invention, the system may also include an attenuator which is positioned on the optical axis of the output laser beam to limit the energy level in pulses of the output laser beam. The computer is electronically connected to the attenuator and, in addition to monitoring the energy level in pulses of the output laser beam, the computer can be used to control this energy level.

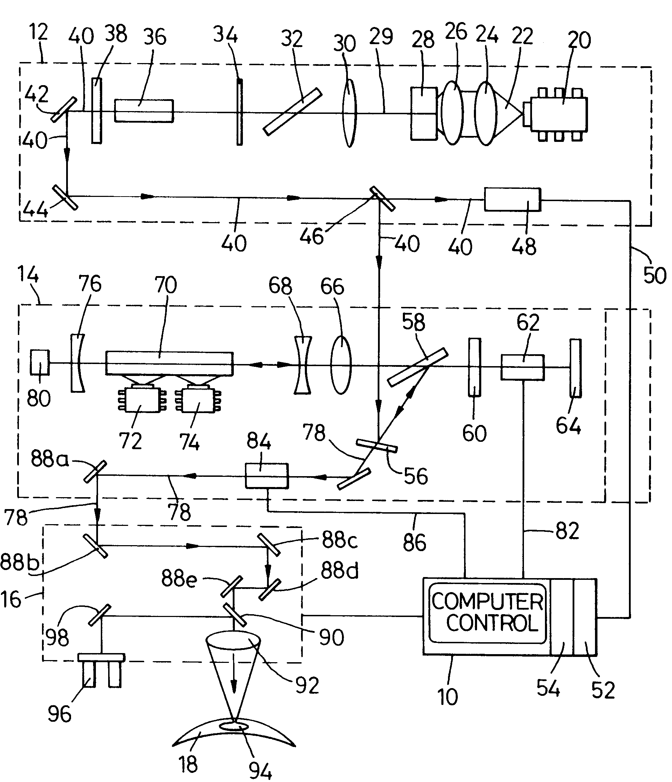

### BRIEF DESCRIPTION OF THE DRAWING

The novel features of this invention, as well as the invention itself, both as to its structure and its operation will be best understood from the accompanying drawings, taken in conjunction with the accompanying description, in which similar reference characters refer to similar parts, and in which:

The Figure is a schematic block diagram of the component elements of the computer-controlled variable pulse repetition rate laser beam generating system of the present invention.

### DESCRIPTION OF THE PREFERRED EMBODIMENTS

Referring to the Figure, a system for varying the pulse repetition rate of a pulsed laser beam is shown to include a computer 10, an oscillator 12, a regenerative amplifier 14 and a beam delivery guidance apparatus 16. As intended for the present invention, the object of the system is to direct a laser beam into a portion of the eye of a patient, such as the cornea 18, for ophthalmic surgery.

In overview, it is well known that an oscillator 12 is useful for producing a beam of very low energy laser pulses (e.g. twenty milliwatts) at an extremely high pulse repetition rate (e.g. one hundred and sixty million Hertz). Further, it is well known that a regenerative amplifier 14 is useful for amplifying pulses from the oscillator 12 and generating an output beam of these amplified pulses having a lower pulse repetition rate than the pulse repetition rate in the initial beam generated by the oscillator 12. For the present invention, the computer 10 is used to variably select the repetition pulse rate of the output beam.

In the Figure, oscillator 12 is shown to include a laser diode 20 which is used to generate a beam of monochromatic light 22. This beam 22 is then passed through the lenses 24,26 and focused onto an oscillator crystal 28. For the present invention, the oscillator crystal 28 is preferably a Nd:YLF laser crystal of a type well known in the pertinent art. A light.beam 29, average from crystal 28 and then successively passes through an oscillator lens 30, a Brewster's window 32 and an etalon 34. As will be appreciated by the skilled artisan, the Brewster's window 32 can be used to monitor the laser in oscillator 12, and the etalon 34 can be used for limiting the bandwidth of light in laser beam 292 to improve stability.

A mode locker 36 is also included in the oscillator 12 for producing laser pulses of extremely short duration. For the present invention, mode locker 36 may be of any type well known in the pertinent art. An output coupler 38 is used to transfer the initial pulsed laser beam 40 created from light beam 29 by oscillator 12, from oscillator 12 to regenerative amplifier 14. To do this turning mirrors 42, and 44 are used to direct the initial laser beam 40 from output coupler 38 toward beam splitter 46. For the particular embodiment shown in the Figure, beam splitter 46 is then used to direct a portion of beam 40 toward regenerative amplifier 14, and to direct the remaining portion of beam 40 toward a photodiode sensor 48. Specifically, the sensor 48 can be of any type sensor known in the art which is capable of detecting the pulse repetition rate in a pulsed laser beam and generating a signal that is indicative of this pulse repetition rate. Attentively, the pulse repetition rate from oscillator 12 can be ascertained by directly sampling the drive signal of mode locker 36.

For the purposes of further disclosure this initial pulse repetition rate is sometimes referred to as the first repetition rate, and the signal generated by sensor 48 which is indicative of this first pulse repetition rate is sometimes referred to as the first signal. As indicated above, the first repetition rate is on the order of 160 MHz.

As shown, photodiode sensor 48 is connected directly to computer 10 via a connector 50. More specifically, connector 50 transmits this first signal from sensor 48 to the computer 10. Further, as indicated in the Figure, this first signal from sensor 48 is passed through a divide by forty divider 52 and then through a divide by twenty divider 54. Dividers 52 and 54 are of a type well known in the art, and the process of dividing beam 40 with the dividers 52, 54 results in an intermediate signal which is equal to the first pulse repetition rate divided by four hundred. Using 160 MHz as an exemplary magnitude for the first signal, the dividers 52 and 54 establish an intermediate signal at the computer 10 that is indicative of a two hundred kilohertz (200 kHz) repetition rate. It is to be appreciated that if the drive signal from mode locker 36 is used to establish the first signal, then appropriate division must be accomplished to establish the intermediate signal.

The portion of laser beam 40 which is directed from oscillator 12 toward regenerative amplifier 14 by beam splitter 46 is first incident on an injection beam splitter 56. As shown, at the beam splitter 56 part of the beam 40 is reflected toward a polarizer 58. From polarizer 58, beam 40 is passed through a quarter wave plate 60 and directed to a pockels cell 62. According to the activation state of the pockels cell 62, laser pulses in the beam 40 will either be blocked by the pockels cell 62 or selectively admitted by the pockels cell 62 into the cavity of regenerative amplifier 14 for amplification. If admitted, the pulse will be reflected by a flat end mirror 64 for passage back through pockels cell 62, quarterwave plate 60 and polarizer 58. The admitted pulse then passes through a positive regenerative amplifier lens 66 and a negative regenerative amplifier lens 68 for proper beam diameter adjustment before being passed through a laser rod 70.

Laser rod 70 is preferably a laser medium such as Nd:YLF which is pumped by laser diodes 72 and 74 to provide a reservoir of electrons which have been elevated to a high energy state. In a manner well known in the pertinent art, as an admitted pulse passes through the laser rod 70, the energy from the electrons is transferred as photons into the pulse. The pulse is then reflected by a curved end mirror 76 back through the laser rod 70 and through the other optical components of regenerative amplifier 14. The pulse continues to shuttle back and forth through the regenerative amplifier 14, between flat end mirror 64 and curved end mirror 76, to take energy from the laser rod 70. This continues until the amplified pulse is dumped from the regenerative amplifier 14. Unless the pulse is intentionally dumped from regenerative amplifier 14, the pulse will continue shuttling back and forth through the regenerative amplifier 14 until it has effectively depleted the energy which was pumped into laser rod 70. It has been determined that the highest rate at which pulses can be maximumly energized by a laser rod 70 is approximately four hundred pulses per second. At faster rates (i.e. pulse repetition rates >400 Hz), the pulses will be admitted and dumped before laser rod 70 can be fully regenerated. At slower rates pulse repetition rates <400 Hz), the laser rod 70 has sufficient time to regenerate and the pulses will attain a common maximum energy level. Stated differently, as the pulse repetition rate decreases toward 400 Hz, the energy level in the individual pulses increases. At approximately 400 Hz and below, however, all pulses have approximately the same maximum energy level that is obtainable from laser rod 70.

As stated aboye, after the pulses which are admitted into the regenerative amplifier 14 have been amplified, they are dumped from the regenerative amplifier 14. Dumping, like admitting, is done through the action of the pockels cell 62. Specifically, the pockels cell 62 can be activated to change the polarization of light in the pulse so that the pulse is directed out of the regenerative amplifier 14 by the polarizer 58 as an output laser beam 78.

In actuality, the output laser beam 78 will have a pulse repetition rate which is established by the rate at which the pockels cell 62 admits pulses into the cavity of the regenerative amplifier 14. For purposes of the disclosure of the present invention, the pulse repetition rate of the output laser beam 78 will sometimes be referred to as the second pulse repetition rate, and any electronic signal indicative of the second pulse repetition rate will sometimes be referred to as the second signal.

Between pulses in the output laser beam 78, it is always necessary for the laser rod 70 to regenerate. This regeneration of laser rod 70 is accomplished by the pumping action of the laser diodes 72,74, and it requires time. As implied above, if the second pulse repetition rate is greater than approximately 400 Hz, the laser rod 70 will not regenerate to its maximum potential during the time interval between pulses. On the other hand, for a second pulse repetition rate which is lower than 400 Hz, laser rod 70 can fully regenerate. In either case, it is important that a pulse be dumped from regenerative amplifier 14 as soon as it has extracted the maximum amount of energy obtainable from laser rod 70. This is so in order to give the output pulse the maximum obtainable energy at the particular repetition rate. To accomplish this function, a photodiode 80 is provided.

In operation, photodiode monitors the pulses as they are being amplified in their passes through laser rod 70. When photodiode 80 indicates the pulse has attained its maximum energy level, photodiode 80 triggers pockels cell 62, by a hardware connection not shown, to dump the pulse. Interestingly, it is not the rate at which pulses are dumped from the regenerative amplifier that establishes the pulse repetition rate of the output laser beam 78. Instead, the second pulse repetition rate is actually established by the admission of pulses into the regenerative amplifier 14 by the pockels cell 60.

As stated above, the dividers 52,54 establish an intermediate signal which is approximately equal to a repetition rate of 200 kHz. Using conventional software programs, computer 10 is able to further divide this intermediate signal by integers to obtain a signal which is indicative of a lower repetition rate. In this manner, a second signal which is indicative of the second pulse repetition rate is established as desired by the operator of computer 10. As shown in the Figure, this second signal is transmitted via connector 82 to pockels cell 62 and used to activate pockels cell 62 to admit pulses from laser beam 40 into the regenerative amplifier 14. In sum, computer 10 activates pockels cell 62 to admit pulses into the regenerative amplifier 14, and photodiode 80 activates pockels cell 62 to dump amplified pulses from the regenerative amplifier 14.

The Figure also shows that the output laser beam 78 is passed through an attenuator 84 which is provided to both monitor and control the energy level of the pulses in output laser beam 78. As shown, attenuator 84 is connected by way of connector 86 to the computer 10 which includes software for these purposes. Specifically, because it may be the case that individual pulses have energy levels which are too high for the particular surgical procedure, the computer-controlled attenuator 84 may be activated to reduce the pulse energy levels in output laser beam 78 as desired.

Turning mirrors 88a-e are of types well known in the pertinent art and are used to direct the output laser beam 78 along the desired path. As shown in the Figure, the output laser beam 78 is directed through the turning mirrors 88, a partial mirror 90, and an objective lens 92 of beam delivery apparatus 16. According to the purposes of the present invention, the output laser beam 78 is focussed by objective lens 92 onto the target tissue 94 in cornea 18 for accomplishment of the desired ophthalmic surgical procedure. The Figure also shows that a microscope 96 can be provided with apparatus 16, and that a turning mirror 98 can be used to establish an optical axis from the microscope 96 that is directed by partial mirror 90 toward the cornea 18. Thus, with microscope 96 the ophthalmic surgical laser procedure can be observed by the operator, as desired.

While the particular computer-controlled variable repetition rate laser system as herein shown and disclosed in detail is fully capable of obtaining the objects and providing the advantages herein before stated, it is to be understood that it is merely illustrative of the presently preferred embodiments of the invention and that no limitations are intended to the details of the construction or design herein shown other than as defined in the appended claims.

## Claims

1. A system for varying the pulse repetition rate of a pulsed laser beam which comprises:
an oscillator for generating a beam comprising a plurality of pulses of monochromatic light, said pulses being generated by said oscillator at a first rate;
a computer, electronically connected to said oscillator to monitor said first rate, said computer having means for selectively dividing said first rate to establish a second rate; and
a regenerative amplifier having a pockels cells, said pockels cell being electronically connected to said computer, and activated by said computer to admit pulses from said oscillator into said regenerative amplifier at said selected second rate for generation of an output laser beam having a variable pulse repetition rate according to said selected second rate.

2. A system according to claim 1 wherein said first rate is approximately equal to one hundred sixty megahertz (160 MHz) and said selected second rate is in the range between zero and two hundred kilohertz (0-200 kHz).

3. A system according to claim 1 wherein the pulses duration of each pulse in said output laser beam is less than approximately sixty picoseconds (60 psec).

4. A system for varying the pulse repetition rate of a pulsed laser beam for generation of an output laser beam which comprises:
means for monitoring a laser beam, said beam comprising a plurality of pulses with said pulses in said beam being consecutively separated by a substantially constant first time interval;
means electronically connected to said monitoring means for variably selecting a second time interval between two of said pulses in said beam, said second time interval being an integer factor of said first time interval and in the range of greater than four hundred times said first time interval; and
means electronically controlled by said selected means for receiving pulses from said beam, said pulses being consecutively separated by said second time interval for subsequent amplification of said pulses and generation of said output laser beam.

5. A system according to claim 4 wherein said monitoring means is a photodiode.

6. A system according to claim 4 or 5 wherein said system includes an oscillator having a mode locker activated by a drive signal and said monitoring means is connected to said mode locker to receive said drive signal therefrom.

7. A system according to any one of claims 4 to 6 wherein said selecting means is a computer.

8. A system according to any one of claims 4 to 7 wherein said receiving means is a Pockels cell and said Pockels cell is mounted in a regenerative amplifier.

9. A system according to claim 1 or 8 wherein each said pulse in said output laser beam has an energy level, and said system further comprises an attenuator optically connected to said regenerative amplifier to measure said energy level in each said pulse in said output laser beam, and wherein said computer is electronically connected to said attenuator to control said energy level.

10. A system according to any one of claims 4 to 9 wherein said first time interval corresponds to a rate of approximately one hundred sixty megahertz (160 MHz) and said second time interval corresponds to a rate in the range of approximately less than two hundred kilohertz (200 kHz).

11. A method for varying the pulse repetition rate of a pulsed laser beam which comprises the steps of:
Determining the pulse repetition rate of a laser beam as said beam is initially generated by an oscillator, said laser beam comprising a plurality of pulses of monochromatic light;
Establishing a first signal indicative of said oscillator generated pulse repetition rate;
Transmitting said first signal to a computer;
Dividing said first signal by a preselected integer using electronic means in said computer to establish a second signal; and
Activating a pockels cell with said second signal from said computer to introduce pulses from said oscillator generated pulsed laser beam into a regenerative amplifier to generate an output laser beam having a varied pulse repetition rate established by said second signal.

12. A method according to claim 11 wherein said output laser beam has an energy level and said method further comprises the steps of:
Monitoring said energy level of said output laser beam with said computer; and
Attenuating said energy level with electronic means in said computer to control said energy level.

13. A method according to claim 12 wherein said repetition rate of said oscillator generated pulsed laser beam is approximately one hundred sixty megahertz (160 MHz) and said preselected integer is equal to or greater than four hundred.

14. A method according to claim 12 wherein the pulse duration of each pulse in said output laser beam is less than approximately sixty picoseconds (60 psec).
